# EUROPÄISCHE PATENTANMELDUNG

(11) **EP 4 083 028 A1**
(43) Veröffentlichungstag der Anmeldung: **02.11.2022**
(21) Anmeldenummer: 21171066.0
(22) Anmeldetag: 28.04.2021
(51) Int. Cl.: C07D 307/60

(54) **VERFAHREN ZUR CARBONYLIERUNG VON EPOXIDEN**

(71) Anmelder: Covestro Deutschland AG, 51373 Leverkusen (DE)
(72) Erfinder: Machat, Martin, 50735 Köln (DE); Schumacher, Hannah, 50733 Köln (DE); Wolf, Aurel, 42489 Wülfrath (DE); Langanke, Jens, 53894 Mechernich (DE); Guertler, Christoph, 50735 Köln (DE); Raju, Suresh, 52072 Aachen (DE); Lansing, Markus, 51381 Leverkusen (DE); Mäkiniemi, Roi, 52074 Aachen (DE); Tyagi, Deepika, 52062 Aachen (DE)
(74) Vertreter: Levpat

(57) **Zusammenfassung**

Gegenstand der Erfindung ist ein Verfahren zur Carbonylierung von Epoxiden in Gegenwart von Kohlenmonoxid und eines Katalysatorsystems, wobei das Katalysatorsystem ein komplexes anionisches Carbonylcobaltat und spezielle, kationische Metallkomplexe aufweist sowie die Verwendung erfindungsgemäßer Katalysator-Systeme zur Carbonylierung von Epoxiden. Ein weiterer Erfindungsgegenstand sind ein Carbonylierungsproduktgemisch erhältlich nach dem erfindungsgemäßen Verfahren sowie ein Verfahren zur Herstellung von Carbonylierungsfolgeprodukten durch Umsetzung des erfindungsgemäßen Carbonylierungsproduktgemisches mit Glycolen und/oder Epoxiden.

## Beschreibung

Gegenstand der Erfindung ist ein Verfahren zur Carbonylierung von Epoxiden in Gegenwart von Kohlenmonoxid und eines Katalysatorsystems, wobei das Katalysatorsystem ein komplexes anionisches Carbonylcobaltat und spezielle, kationische Metallkomplexe aufweist sowie die Verwendung erfindungsgemäßer Katalysator-Systeme zur Carbonylierung von Epoxiden. Ein weiterer Erfindungsgegenstand sind das Carbonylierungsproduktgemisch erhältlich nach dem erfindungsgemäßen Verfahren sowie ein Verfahren zur Herstellung von Carbonylierungsfolgeprodukten durch Umsetzung des erfindungsgemäßen Carbonylierungsproduktgemisches mit Glycolen und/oder Epoxiden.

In US 8,481,756 B1 werden Katalysatorsysteme und ein Verfahren zur Doppelcarbonylierung von verschiedenen substituierten und unsubstituierten Epoxiden unter Bildung von cyclischen Anhydriden wie substituierter oder unsubstituierter Bernsteinsäure in unterschiedlichen Lösungsmitteln offenbart, wobei die Katalysatorsysteme eine Lewis-Säure-Spezies und einen Übergangsmetallcarbonyl-Komplex umfasst. Hierbei erfolgt die Carbonylierungsreaktion in einem Einschrittverfahren, ohne dass die Lacton-Zwischenstufe isoliert und gereinigt werden muss. In den Ausführungsbeispielen von US 8,481,756 B1 aber auch in Rowley et al. J. Am. Chem. Soc. 2007, 129, 4948-4960 werden als Katalysatorsysteme ein Aluminium- oder ein Chrom-Porphyrinkomplex oder ein Aluminium-Salphenkomplex als kationische Lewis-Säure Spezies jeweils neben dem anionischen Tetracarbonylcobalt-Komplex verwendet. Beispielsweise erfolgt die Doppelcarbonylierung von Ethylenoxid und Propylenoxid bei 90 °C für 2-3 Stunden, um vollständige Umsätze und hohe Ausbeuten des cyclischen Anhydrids zu erzielen. Hierbei weisen die Aluminium- oder Chrom-Porphyrinkomplexe eine höhere Aktivität als die Aluminium-Salphenkomplexe auf.

Hierbei werden jedoch geringe Konzentrationen (1.0-2.0 M) des Epoxids im Lösungsmittel eingesetzt. Weiterhin sind die verwendeten Aluminium- oder ein kationischer Chrom-Porphyrinkomplex oder ein Aluminium-Salphenkomplex nur sehr bedingt luft- bzw. feuchtigkeitsstabil, was eine ausschließliche Handhabung dieser Systeme unter inerten Bedingungen erfordert.

Ausgehend vom Stand der Technik war es Aufgabe der vorliegenden Erfindung die Nachteile von kationischen Porphyrinkomplexen für Cobalt-basierte Katalysatorsysteme für die Carbonylierung von Epoxiden zu reduzieren.

Es war daher die Aufgabe der vorliegenden Erfindung, ein einfaches Verfahren für die Carbonylierung von Epoxiden zu Carbonylierungsprodukten unter Einsatz eines Katalysatorsystems bereitzustellen, wobei im Speziellen beta-Lactone durch Einfachcarbonylierung von Epoxiden und cyclische Anhydride durch Doppelcarbonylierung von Epoxiden mithilfe des erfindungsgemäßen Verfahren bereitgestellt werden sollen.

Hierbei soll ein chemisch stabileres Katalysatorsystem verwendet werden, so dass eine längere Lagerung und/oder eine technisch einfachere Lagerung, Handling und Nutzung auch bei kurzzeitiger Exposition dieser Katalysatorsysteme an Luft und/oder Luftfeuchtigkeit gegenüber aus dem Stand der Technik vorbeschriebenen Porphyrinkomplex- oder Salphenkomplex-basierten Katalysatorsystemen möglich ist. Weiterhin soll auch ein Verfahren bereitgestellt werden, bei dem die Konzentration des eigesetzten Epoxids im Lösungsmittel bei weiterhin guter Selektivität zu den cyclischen Carbonylierungsprodukten wie beispielsweise Lacton und/oder cyclisches Anhydrid erhöht werden kann, so dass weniger Lösungsmittel aus den resultierenden Carbonylierungsprodukten, speziell aus den cyclischen Anhydriden nach der Doppelcarbonylierung in einem technisch aufwendigen Destillationsschritt abgetrennt werden muss. Außerdem soll auch ein Katalysatorsystem bzw. ein Verfahren mit verbesserter Raum-Zeit-Ausbeute zur Verfügung gestellt werden, was die Durchführung der Carbonylierung bei höheren Temperaturen erlaubt, da höhere Temperaturen im Carbonylierungsverfahren auch eine verbesserte Löslichkeit der eingesetzten Epoxide bzw. der Carbonylierungsprodukte wie beispielsweise der cyclischen Anhydride bewirken, so dass eine ungewollte Kristallisation dieser Carbonylierungsprodukte im Reaktor vermieden werden kann.

Überraschend wurde gefunden, dass die erfindungsgemäße Aufgabe gelöst wird durch ein Verfahren zur Carbonylierung von Epoxiden in Gegenwart von Kohlenmonoxid und eines Katalysatorsystems,
wobei das Katalysatorsystem eine Struktur der Formel (I) aufweist: 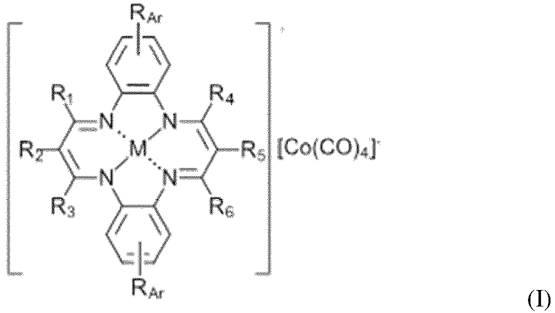 wobei M Al, Cr, Fe, Mo, W, Sc, Y, Bi, Ru, Rh, La, Ce, Nd, Sm, In oder Ga ist, wobei M bevorzugt Al oder Cr ist,
wobei R_{Ar}: an jeder Position unabhängig voneinander ausgewählt aus der Gruppe von: H, C1-C12-Alkyl, C2-C12-Alkenyl, C2-C12-Alkinyl, O-(C1-C12-Alkyl), O-(C2-C12-Alkenyl), O-(C2-C12-Alkinyl), O-CO-(C1-C12-Alkyl), O-CO-(C2-C12-Alkenyl), O-CO-(C2-C12-Alkinyl), -CH2-O-(C1-C12-Alkyl), -CH2-O-(C2-C12-Alkenyl), -CH2-O-(C2-C12-Alkinyl), -CO-O-(C1-C12-Alkyl),-CO-O-(C2-C12-Alkenyl), -CO-O-(C2-C12-Alkinyl), -CF₃, -OCF₃, bevorzugt H, Methyl, Ethyl, n-Propyl, iso-Propyl, n-Butyl-, iso-Butyl, tert-Butyl, O-Methyl, O-Ethyl, O-n-Propyl, O-iso-Propyl, O-n-Butyl-, O-iso-Butyl, O-tert-Butyl, besonders bevorzugt H oder Methyl,
wobei R₁, R₃, R₄, R₆ an jeder Position unabhängig voneinander ausgewählt aus der Gruppe von H, C1-C12-Alkyl, C2-C12-Alkenyl, C2-C12-Alkinyl, Aryl (C6R'5; R' an jeder Position unabhängig voneinander gewählt aus der Gruppe von H, C1-C12-Alkyl), Phenyl, Benzyl, Cyclohexyl (C6R'11; R' an jeder Position unabhängig voneinander gewählt aus der Gruppe von H, C1-C12-Alkyl), -CF₃, bevorzugt Methyl, Ethyl, , n-Propyl, iso-Propyl, n-Butyl-, iso-Butyl, tert-Butyl, -CF₃, Phenyl, besonders bevorzugt Methyl und tert-Butyl,
wobei R₂, R₅ an jeder Position unabhängig voneinander ausgewählt aus der Gruppe von H, C1-C12-Alkyl, C2-C12-Alkenyl, C2-C12-Alkinyl, Aryl (C6R'5; R' an jeder Position unabhängig voneinander gewählt aus der Gruppe von H, C1-C12-Alkyl), Phenyl, Benzyl, Cyclohexyl (C6R'11; R' an jeder Position unabhängig voneinander gewählt aus der Gruppe von H, C1-C12-Alkyl), -CF₃, bevorzugt H, Methyl, Ethyl, , n-Propyl, iso-Propyl, n-Butyl-, iso-Butyl, tert-Butyl, -CF₃, Phenyl, besonders bevorzugt H,
wobei die Carbonylierung bei Temperaturen von 40 °C bis 130 °C, bevorzugt von 45 °C bis 125 °C und besonders bevorzugt von 50 °C bis 120 °C durchgeführt wird,
wobei die Carbonylierung folgende Schritte umfasst
i) Umsetzung des Epoxids mit Kohlenmonoxid bei einer ersten Temperatur T(i) zu einem Lacton-haltigen Intermediat-Gemisch (i)
ii) Umsetzung des Lacton-haltigen Intermediat-Gemischs (i) mit Kohlenmonoxid bei einer zweiten Temperatur T(ii) zum Produktgemisch,
wobei T(ii) > T(i) ist.

Unter dem Lacton-haltigen Intermediat-Gemisch (i) ist erfindungsgemäß ein Umsetzungsprodukt der Carbonylierung von Epoxiden mit einem Lactonanteil wie beispielsweise beta-Butyrolacton (Einfachcarbonylierung von Propylenoxid) oder Propiolacton (Einfachcarbonylierung von Ethylenoxid) von mindestens 50 mol-% zu verstehen, wobei der verbleibende Rest das cyclische Anhydrid als Produkt der Doppelcarbonylierung aber auch Neben- oder Umlagerungsprodukte ist. Hierbei resultieren sowohl molekulare als auch polymere Nebenprodukte (polymere Carbonylierungsprodukte), wobei der Anteil der Nebenprodukte bzw. der Umlagerungsprodukte ≤ 10 mol-% bzw. ≤ 16mol% für das Lacton-haltige Intermediat-Gemisch (i) beträgt. Unter Neben-bzw. Umlagerungsprodukten sind erfindungsgemäß Produkte durch ungewünschte Neben- oder Folgereaktionen zu verstehen, wie beispielsweise Acrylsäure, Crotonsäure, Aceton bzw. Acetaldehyd (Ethanal), Propanal, als Beispiel für molekulare Neben- bzw. Umlagerungsprodukte aber auch Polyether oder Polyester (z.B. Polyhydroxybutyrat, Polyhydroxypropionat) als polymere Nebenprodukte. Der als Stoffmengenanteil polymerer Nebenprodukte angegebene Anteil bezeichnet die Stoffmenge an eingebauten Monomeren (Epoxid oder Lacton) in der Gesamtmenge polymerer Nebenprodukte.

Erfindungsgemäß ist unter dem Produktgemisch (Carbonylierungsproduktgemisch) eine Zusammensetzung mit einem Anteil von 40,0 mol-% bis 98,7 mol-% an cyclischen Anhydrid, ≤ 6 mol-% an polymeren Nebenprodukten, sowie ≤ 5 mol-%, Umlagerungsprodukten sowie ≤ 55 mol-% Lacton zu verstehen.

Das erfindungsgemäße Epoxid kann ein Epoxid mit 2-45 Kohlenstoffatomen sein. In einer bevorzugten Ausführungsform des Verfahrens wird das Epoxid ausgewählt aus mindestens einer Verbindung der Gruppe bestehend aus Ethylenoxid, Propylenoxid, 1,2-Butylenoxid, 2,3-Butylenoxid, 2-Methyl-1,2-propylenoxid (Isobutenoxid), 1,2-Pentylenoxid, 2,3-Pentylenoxid, 2-Methyl-1,2-butylenoxid, 3-Methyl-1,2-butylenoxid, Epoxide von C6-C22 α-Olefinen, wie 1,2-Hexylenoxid, 2,3-Hexylenoxid, 3,4-Hexylenoxid, 2-Methyl-1,2-pentylenoxid, 4-Methyl-1,2-pentylenoxid, 2-Ethyl-1,2-butylenoxid, 1,2-Heptylenoxid, 1,2-Octylenoxid, 1,2-Nonylenoxid, 1,2-Decylenoxid, 1,2-Undecylenoxid, 1,2-Dodecylenoxid, 4-Methyl-1,2-pentylenoxid, Cyclopentylenoxid, Cyclohexylenoxid, Cycloheptylenoxid, Cyclooctylenoxid, Styroloxid, Methylstyroloxid, Pinenoxid, Allylglycedylether, Vinylcyclohexylenoxid, Cyclooctadienmonoepoxid, Cyclododecatrienmono-epoxid, Butadienmonoepoxid, Isoprenmonoepoxid, Limonenoxid, 1,4-Divinylbenzolmonoepoxid, 1,3-Divinylbenzolmonoepoxid, Glycidylacrylat und Glycidylmethacrylat ein- oder mehrfach epoxidierte Fette als Mono-, Di- und Triglyceride, epoxidierte Fettsäuren, C1-C24-Ester epoxidierter Fettsäuren, Epichlorhydrin, Glycidol, und Derivate des Glycidols wie beispielsweise Glycidylether von C1-C22 Alkanolen, Glycidylester von C1-C22 Alkancarbonsäuren. Beispiele für Derivate des Glycidols sind Phenylglycidylether, Kresylglycidylether, Methylglycidylether, Ethylglycidylether und 2-Ethylhexylglycidylether.

In einer bevorzugten Ausführungsform des Verfahrens ist das Epoxid Ethylenoxid, Propylenoxid, 1,2-Butylenoxid, 1,2-Pentylenoxid, 1,2-Hexylenoxid, 1,2-Heptylenoxid und/oder 1,2-Octylenoxid. In einer besonders bevorzugten Ausführungsform des Verfahrens ist das Epoxid Ethylenoxid und/oder Propylenoxid.

In einer Ausführungsform des erfindungsgemäßen Verfahrens weist das verwendete Kohlenmonoxid einen Anteil an Sauerstoff von weniger als 50 ppm, bevorzugt weniger als 40 ppm und besonders bevorzugt von weniger als 20 ppm auf.

In einer Ausführungsform des erfindungsgemäßen Verfahrens ist M in Formel (I) Aluminium (Al).

In einer Ausführungsform des erfindungsgemäßen Verfahrens weist das Katalysatorsystem Strukturen der Formel (II), (III), (IV), (V), (VI) und/oder (VII) auf: 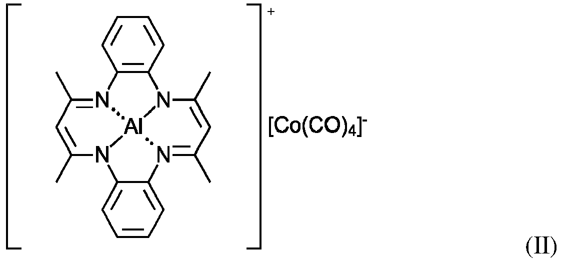 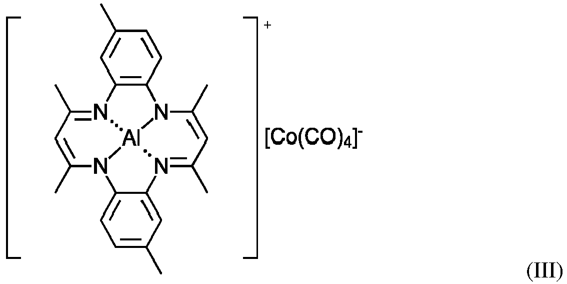 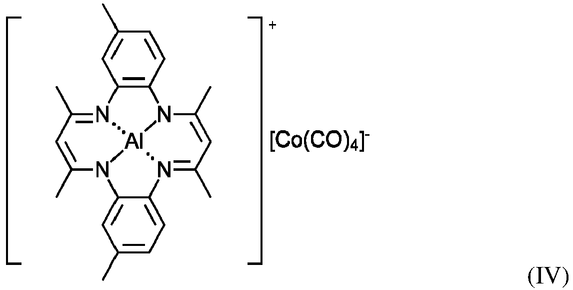 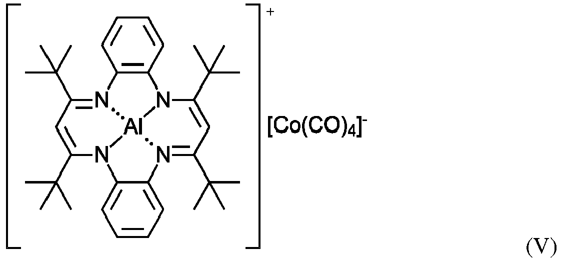 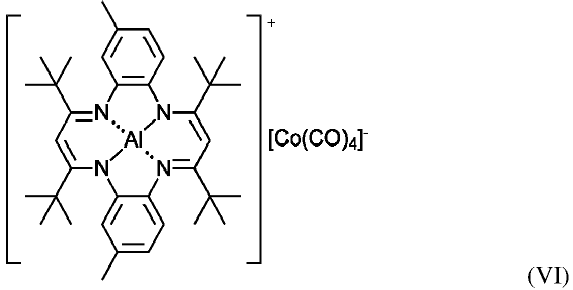 und/oder

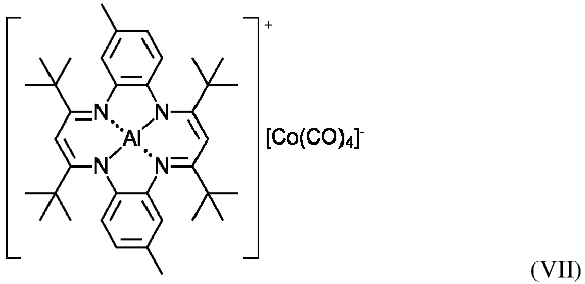

In einer Ausführungsform des erfindungsgemäßen Verfahrens wird das Katalysatorsystem in Mengen von 0,10 mol-% bis 10,00 mol-% bevorzugt von 0,25 mol-% bis 5,00 mol-% und besonders bevorzugt von 0,50 mol-% bis 2,00 mol-% bezogen auf das Epoxid eingesetzt.

In einer Ausführungsform des erfindungsgemäßen Verfahrens wird die Carbonylierung bei Temperaturen T(i) von 40 °C bis 80 °C und T(ii) größer 80°C bis 130 °C, bevorzugt bei T(i) von 45 °C bis 80 °C und T(ii) größer 80°C bis 125 °C und besonders bevorzugt bei T(i) von 50 °C bis 80 °C und T(ii) größer 80°C bis 120 °C durchgeführt.

In einer Ausführungsform des erfindungsgemäßen Verfahrens wird die Carbonylierung bei Drücken von 1 bara bis 120 bara bevorzugt von 10 bara bis 100 bara und besonders bevorzugt von 20 bara bis 80 bara durchgeführt, wobei Schritt i) bei einem Druck p(i) und Schritt ii) bei einem Druck p(ii) durchgeführt wird, wobei p(i) bevorzugt größer als p(ii) ist.

In einer Ausführungsform des erfindungsgemäßen Verfahrens wird Schritt i) bei einem Druck von 1 bara bis 120 bara, bevorzugt von 10 bara bis 100 bara und besonders bevorzugt von 20 bara bis 80 bara durchgeführt.

In einer Ausführungsform des erfindungsgemäßen Verfahrens wird Schritt ii) bei einem Druck von 1 bara bis 120 bara, bevorzugt von 5 bara bis 100 bara und besonders bevorzugt von 10 bara bis 80 bara durchgeführt.

In einer Ausführungsform des erfindungsgemäßen Verfahrens wird Schritt i) für 10 min bis 12 h bevorzugt für 30 min bis 10 h und besonders bevorzugt für 60 min bis 8 h durchgeführt.

In einer Ausführungsform des erfindungsgemäßen Verfahrens wird Schritt ii) für 10 min bis 12 h bevorzugt für 30 min bis 9 h und besonders bevorzugt für 60 min bis 6 h durchgeführt.

In einer Ausführungsform des erfindungsgemäßen Verfahrens weist das Lacton-haltige Intermediat-Gemisch (i) eine Selektivität zur Bildung des Lactons von 50 % bis 100 %, bevorzugt von 50 % bis 95 % und besonders bevorzugt von 60 % bis 90 % auf.

Erfindungsgemäß ist unter einem Lösungsmittel eine oder mehrere Verbindungen zu verstehen, welche das Epoxid teilweise, bevorzugt vollständig löst. Auch die erfindungsgemäßen Katalysatorsysteme sind mindestens teilweise unter Reaktionsbedingungen in dem Lösungsmittel löslich.

In einer Ausführungsform des erfindungsgemäßen Verfahrens wird die Carbonylierung in einem Lösungsmittels, bevorzugt einem aprotischen Lösungsmittel durchgeführt.

Die erfindungsgemäß eingesetzten Lösungsmittel enthalten keine H-funktionellen Gruppen. Als Lösungsmittel sind alle polar-aprotischen, schwach polar-aprotischen und unpolar-aprotischen Lösungsmittel geeignet, die jeweils keine H-funktionellen Gruppen enthalten. Als Lösungsmittel können auch eine Mischung aus zwei oder mehrerer dieser Lösungsmittel eingesetzt werden. Beispielhaft seien an dieser Stelle folgende polar-aprotischen Lösungsmittel genannt: 4-Methyl-2-oxo-1,3-dioxolan (im Folgenden auch als cyclisches Propylencarbonat oder cPC bezeichnet), 1,3-Dioxolan-2-on (im Folgenden auch als cyclisches Ethylencarbonat oder cEC bezeichnet), Methylformiat, Ethylformiat, Isopropylformiat, Propylformiat, Ethylacetat, Isopropylacetat, n-Butylacetat, Oxalsäuremethylester, 2,2-Diemthoxypropan, Aceton, Methylethylketon, Acetonitril, Benzonitril, Diethylcarbonat, Diemethylcarbonat, Nitromethan, Dimethylsulfoxid, Sulfolan, Dimethylformamid, Dimethylacetamid und *N*-Methylpyrrolidon.

Zu der Gruppe der unpolar- und schwach polar-aprotischen Lösungsmittel zählen z.B. Ether, wie z.B. Dioxolan, Dioxan, Diethylether, Methyl-tert-Butylether, Ethyl-tert-Butylether, tert-Amylmethylether, Butylmethylether, Methylpropylether, Dimethylether, Diisopropylether, Ethylmethylether, Methylphenylether, Dimethoxymethan, Diethoxymethan, Dimethoxyethan (Glyme), [12]Krone-4, Cyclopentylmethylether, Triglyme, Tetraglyme, Diethylenglycoldibutylether, 2-Methyltetrahydrofuran und Tetrahydrofuran, Ester, wie z.B. Essigsäureethylester und Essigsäurebutylester, Kohlenwasserstoffe, wie z.B. Pentan, n-Hexan, Benzol und alkylierte Benzolderivate (z.B. Toluol, Xylol, Ethylbenzol) und chlorierte Kohlenwasserstoffe, wie z.B., Chloroform, Chlorbenzol, Dichlorbenzol, Fluorbenzol, Difluorbenzol, Methylenechlorid, und Tetrachlorkohlenstoff.

In einer bevorzugten Ausführungsform des erfindungsgemäßen Verfahrens ist das Lösungsmittel eine oder mehrere Verbindung(en) und wird ausgewählt aus der Gruppe bestehend aus 4-Methyl-2-oxo-1,3-dioxolan, 1,3-Dioxolan-2-on, 2-Methyltetrahydrofuran, Tetrahydrofuran, 1,4-Dioxan, Dimethoxyethan (Glyme), Bis(2-methoxyethyl)ether (Diglyme), Tris(2-methoxyethyl)ether (Triglyme), Diethylether, Ethylacetat, Methylethylketon, *N*-Methylpyrrolidon, Acetonitril, Sulfolan, Dimethylsulfoxid, Dimethylformamid, Hexan, Toluol, Xylol, Ethylbenzol, Dichlorbenzol, und Chlorbenzol, bevorzugt 4-Methyl-2-oxo-1,3-dioxolan, 1,3-Dioxolan-2-on, 2-Methyltetrahydrofuran, Tetrahydrofuran, 1,4-Dioxan, Dimethoxyethan (Glyme), Bis(2-methoxyethyl)ether (Diglyme), Diethylether, Ethylacetat, Acetonitril, Toluol, Dichlorbenzol und Chlorbenzol, besonders bevorzugt Tetrahydrofuran.

In einer Ausführungsform des erfindungsgemäßen Verfahrens beträgt die Konzentration des Epoxids im Lösungsmittel von 0,5 mol/L bis 10,0 mol/L, bevorzugt von 1,0 mol/L bis 9,0 mol/L und besonders bevorzugt von 2,0 mol/L bis 8,0 mol/L.

Ein weiterer Gegenstand der vorliegenden Erfindung ist ein Carbonylierungsproduktgemisch erhältlich nach dem erfindungsgemäßen Verfahren, wobei der Anteil an cyclischem Anhydrid weniger als 99,0 mol-% bevorzugt von 80,0 bis 98,8 mol-% sowie der Anteil an polymeren Carbonylierungsprodukten weniger als 7,0 mol-% bevorzugt weniger als 2,0 mol-% beträgt, wobei die Stoffmengenanteile des cyclischen Anhydrids sowie der polymeren Carbonylierungsprodukte mittels der im Experimentalteil offenbarten Methode ¹H NMR Spektroskopie bestimmt wurde.

Die polymeren Carbonylierungsprodukte wie beispielsweise Polyhydroxybutyrat (PHB) und Polyhydroxypropionat (PHP) oder deren Mischungen sind hierbei (Co)Polymerisationsprodukte aus 4-Ringlactonen wie beispielsweise *beta*-Butyrolacton und/oder Propiolacton. Diese 4-Ringlactone wie *beta*-Butyrolacton oder Propiolacton sind durch Einfachcarbonylierung von Epoxiden wie beispielsweise Propylenoxid oder Ethylenoxid mit Kohlenmonoxid erhältlich.

Die polymeren Carbonylierungsprodukte weisen neben Polyester-Wiederholungseinheiten (resultierend aus Lacton als cyclischer Ester) auch Polyether-Wiederholungeinheiten beispielswiese infolge der Insertion von Alkylenoxiden auf.

Hierbei kann Polyhydroxybutyrat 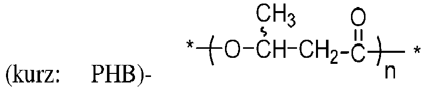 und/oder Polyhydroxypropionat 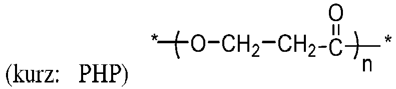 in Form von Co-Polymeren zusammen mit Polyether-Wiederholungseinheiten, 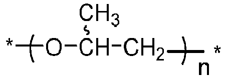 wie z.B. Polyether-Wiederholungseinheit gebildet aus PO und/oder 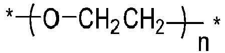 Polyether-Wiederholungseinheit gebildet aus EO, vorliegen (* = Endgruppen und/oder andere Wiederholungseinheiten).

Ein weiterer Gegenstand der vorliegenden Erfindung ist ein Verfahren zur Herstellung von Carbonylierungsfolgeprodukten, durch Umsetzung des erfindungsgemäßen Carbonylierungsproduktgemisches mit Glycolen und/oder Epoxiden.

Hierbei entspricht die Definition der Epoxide derer des Carbonylierungsverfahrens.

Auch die Verwendung des erfindungsgemäßen Katalysatorsystems für die Carbonylierung von Epoxiden ist ein Gegenstand der vorliegenden Erfindung.

In einer ersten Ausführungsform betrifft die Erfindung ein Verfahren zur Carbonylierung von Epoxiden in Gegenwart von Kohlenmonoxid und eines Katalysatorsystems,
wobei das Katalysatorsystem eine Struktur der Formel (I) aufweist: 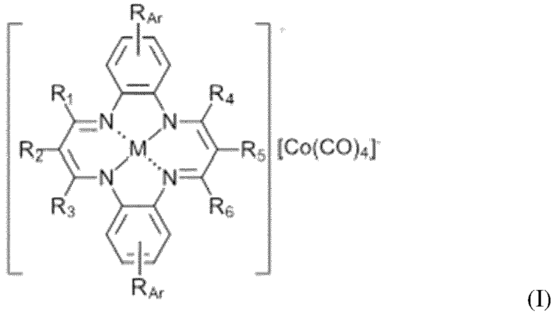 wobei M Al, Cr, Fe, Mo, W, Sc, Y, Bi, Ru, Rh, La, Ce, Nd, Sm, In oder Ga ist, wobei M bevorzugt Al oder Cr ist,
wobei R_{Ar}: an jeder Position unabhängig voneinander ausgewählt aus der Gruppe von: H, C1-C12-Alkyl, C2-C12-Alkenyl, C2-C12-Alkinyl, O-(C1-C12-Alkyl), O-(C2-C12-Alkenyl), O-(C2-C12-Alkinyl), O-CO-(C1-C12-Alkyl), O-CO-(C2-C12-Alkenyl), O-CO-(C2-C12-Alkinyl), -CH2-O-(C1-C12-Alkyl), -CH2-O-(C2-C12-Alkenyl), -CH2-O-(C2-C12-Alkinyl), -CO-O-(C1-C12-Alkyl),-CO-O-(C2-C12-Alkenyl), -CO-O-(C2-C12-Alkinyl), -CF₃, -OCF₃, bevorzugt H, Methyl, Ethyl, n-Propyl, iso-Propyl, n-Butyl-, iso-Butyl, tert-Butyl, O-Methyl, O-Ethyl, O-n-Propyl, O-iso-Propyl, O-n-Butyl-, O-iso-Butyl, O-tert-Butyl, besonders bevorzugt H oder Methyl,
wobei R₁, R₃, R₄, R₆ an jeder Position unabhängig voneinander ausgewählt aus der Gruppe von H, C1-C12-Alkyl, C2-C12-Alkenyl, C2-C12-Alkinyl, Aryl (C6R'5; R' an jeder Position unabhängig voneinander gewählt aus der Gruppe von H, C1-C12-Alkyl), Phenyl, Benzyl, Cyclohexyl (C6R'11; R' an jeder Position unabhängig voneinander gewählt aus der Gruppe von H, C1-C12-Alkyl), -CF₃, bevorzugt Methyl, Ethyl, , n-Propyl, iso-Propyl, n-Butyl-, iso-Butyl, tert-Butyl, -CF₃, Phenyl, besonders bevorzugt Methyl und tert-Butyl,
wobei R₂, R₅ an jeder Position unabhängig voneinander ausgewählt aus der Gruppe von H, C1-C12-Alkyl, C2-C12-Alkenyl, C2-C12-Alkinyl, Aryl (C6R'5; R' an jeder Position unabhängig voneinander gewählt aus der Gruppe von H, C1-C12-Alkyl), Phenyl, Benzyl, Cyclohexyl (C6R'11; R' an jeder Position unabhängig voneinander gewählt aus der Gruppe von H, C1-C12-Alkyl), -CF₃, bevorzugt H, Methyl, Ethyl, , n-Propyl, iso-Propyl, n-Butyl-, iso-Butyl, tert-Butyl, -CF₃, Phenyl, besonders bevorzugt H,
wobei die Carbonylierung bei Temperaturen von 40 °C bis 130 °C, bevorzugt von 45 °C bis 125 °C und besonders bevorzugt von 50 °C bis 120 °C durchgeführt wird,
wobei die Carbonylierung folgende Schritte umfasst
i) Umsetzung des Epoxids mit Kohlenmonoxid bei einer ersten Temperatur T(i) zu einem Lacton-haltigen Intermediat-Gemisch (i)
ii) Umsetzung des Lacton-haltigen Intermediat-Gemischs (i) mit Kohlenmonoxid bei einer zweiten Temperatur T(ii) zum Produktgemisch,
wobei T(ii) > T(i) ist.

In einer zweiten Ausführungsform betrifft die Erfindung ein Verfahren gemäß der ersten Ausführungsform, das Epoxid ausgewählt aus mindestens einer Verbindung der Gruppe bestehend aus Ethylenoxid, Propylenoxid, 1,2-Butylenoxid, 2,3-Butylenoxid, 2-Methyl-1,2-propylenoxid (Isobutenoxid), 1,2-Pentylenoxid, 2,3-Pentylenoxid, 2-Methyl-1,2-butylenoxid, 3-Methyl-1,2-butylenoxid, Epoxide von C6-C22 α-Olefinen, wie 1,2-Hexylenoxid, 2,3-Hexylenoxid, 3,4-Hexylenoxid, 2-Methyl-1,2-pentylenoxid, 4-Methyl-1,2-pentylenoxid, 2-Ethyl-1,2-butylenoxid, 1,2-Heptylenoxid, 1,2-Octylenoxid, 1,2-Nonylenoxid, 1,2-Decylenoxid, 1,2-Undecylenoxid, 1,2-Dodecylenoxid, 4-Methyl-1,2-pentylenoxid, Cyclopentylenoxid, Cyclohexylenoxid, Cycloheptylenoxid, Cyclooctylenoxid, Styroloxid, Methylstyroloxid, Pinenoxid, Allylglycedylether, Vinylcyclohexylenoxid, Cyclooctadienmonoepoxid, Cyclododecatrienmono-epoxid, Butadienmonoepoxid, Isoprenmonoepoxid, Limonenoxid, 1,4-Divinylbenzolmonoepoxid, 1,3-Divinylbenzolmonoepoxid, Glycidylacrylat und Glycidylmethacrylat ein- oder mehrfach epoxidierte Fette als Mono-, Di- und Triglyceride, epoxidierte Fettsäuren, C1-C24-Ester epoxidierter Fettsäuren, Epichlorhydrin, Glycidol, und Derivate des Glycidols wie beispielsweise Glycidylether von C1-C22 Alkanolen, Glycidylester von C1-C22 Alkancarbonsäuren. Beispiele für Derivate des Glycidols sind Phenylglycidylether, Kresylglycidylether, Methylglycidylether, Ethylglycidylether und 2-Ethylhexylglycidylether.

In einer dritten Ausführungsform betrifft die Erfindung ein Verfahren gemäß der zweiten Ausführungsform, wobei das Epoxid Ethylenoxid, Propylenoxid, 1,2-Butylenoxid, 1,2-Pentylenoxid, 1,2-Hexylenoxid, 1,2-Heptylenoxid und/oder 1,2-Octylenoxid, bevorzugt Ethylenoxid und/oder Propylenoxid ist.

In einer vierten Ausführungsform betrifft die Erfindung ein Verfahren gemäß einer der ersten bis dritten Ausführungsform, wobei das verwendete Kohlenmonoxid einen Anteil an Sauerstoff von weniger als 50 ppm, bevorzugt weniger als 40 ppm und besonders bevorzugt von weniger als 20 ppm aufweist.

In einer fünften Ausführungsform betrifft die Erfindung ein Verfahren gemäß einer der ersten bis vierten Ausführungsform, wobei M in Formel (I) Aluminium (Al) ist.

In einer sechsten Ausführungsform betrifft die Erfindung ein Verfahren gemäß einer der ersten bis fünften Ausführungsform, wobei das Katalysatorsystem Strukturen der Formel (II), (III), (IV), (V), (VI) und/oder (VII) aufweist: 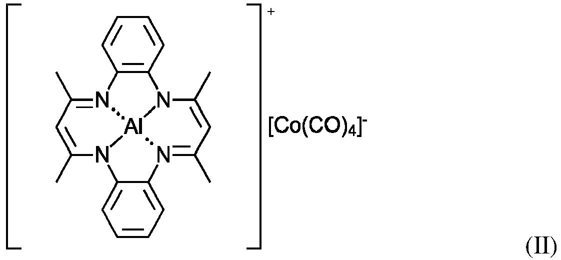 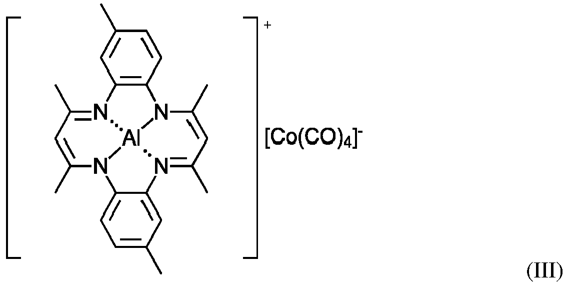 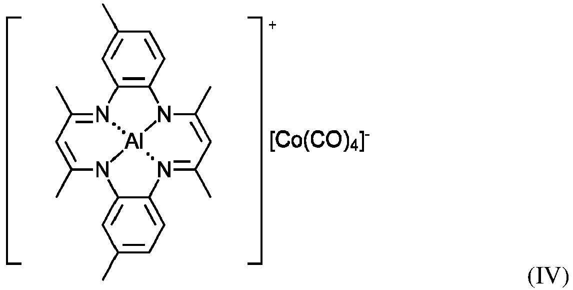 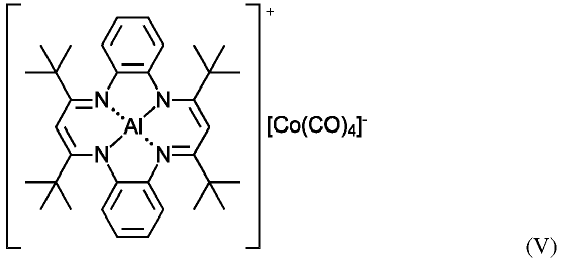 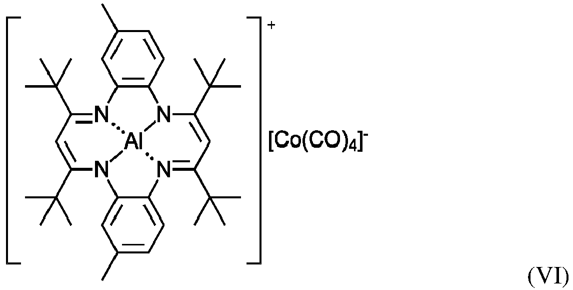 und/oder

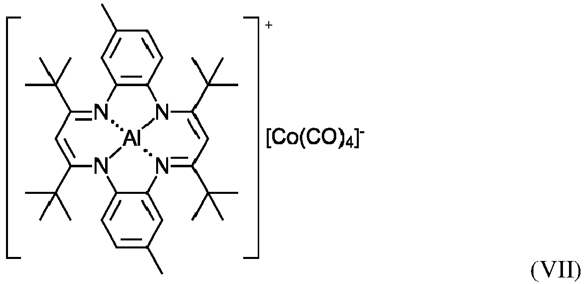

In einer siebten Ausführungsform betrifft die Erfindung ein Verfahren gemäß einer der ersten bis sechsten Ausführungsform, wobei das Katalysatorsystem in Mengen von 0,10 mol-% bis 10,00 mol-% bevorzugt von 0,25 mol-% bis 5,00 mol-% und besonders bevorzugt von 0,50 mol-% bis 2,00 mol-% bezogen auf das Epoxid eingesetzt.

In einer achten Ausführungsform betrifft die Erfindung ein Verfahren gemäß einer der ersten bis siebten Ausführungsform, wobei die Carbonylierung bei Temperaturen T(i) von 40 °C bis 80 °C und T(ii) größer 80°C bis 130 °C, bevorzugt bei T(i) von 45 °C bis 80 °C und T(ii) größer 80°C bis 125 °C und besonders bevorzugt bei T(i) von 50 °C bis 80 °C und T(ii) größer 80°C bis 120 °C durchgeführt wird.

In einer neunten Ausführungsform betrifft die Erfindung ein Verfahren gemäß einer der ersten bis achten Ausführungsform, wobei die Carbonylierung bei Drücken von 1 bara bis 120 bara bevorzugt von 10 bara bis 100 bara und besonders bevorzugt von 20 bara bis 80 bara durchgeführt, wobei Schritt i) bei einem Druck p(i) und Schritt ii) bei einem Druck p(ii) durchgeführt wird, wobei p(i) bevorzugt größer als p(ii) ist.

In einer zehnten Ausführungsform betrifft die Erfindung ein Verfahren gemäß einer der ersten bis neunten Ausführungsform, wobei Schritt i) bei einem Druck von 1 bara bis 120 bara, bevorzugt von 10 bara bis 100 bara und besonders bevorzugt von 20 bara bis 80 bara durchgeführt wird.

In einer elften Ausführungsform betrifft die Erfindung ein Verfahren gemäß einer der ersten bis zehnten Ausführungsform, wobei Schritt ii) bei einem Druck von 1 bara bis 120 bara, bevorzugt von 5 bara bis 100 bara und besonders bevorzugt von 10 bara bis 80 bara durchgeführt wird.

In einer zwölften Ausführungsform betrifft die Erfindung ein Verfahren gemäß einer der ersten bis elften Ausführungsform, wobei Schritt i) für 10 min bis 12 h bevorzugt für 30 min bis 10 h und besonders bevorzugt für 60 min bis 8 h durchgeführt wird.

In einer dreizehnten Ausführungsform betrifft die Erfindung ein Verfahren gemäß einer der ersten bis zwölften Ausführungsform, wobei Schritt ii) für 10 min bis 12 h bevorzugt für 30 min bis 9 h und besonders bevorzugt für 60 min bis 6 h durchgeführt wird.

In einer vierzehnten Ausführungsform betrifft die Erfindung ein Verfahren gemäß einer der ersten bis dreizehnten Ausführungsform, wobei das Lacton-haltige Intermediat-Gemisch (i) eine Selektivität zur Bildung des Lactons von 50 % bis 100 %, bevorzugt von 50 % bis 95 % und besonders bevorzugt von 60 % bis 90 % aufweist.

In einer fünfzehnten Ausführungsform betrifft die Erfindung ein Verfahren gemäß einer der ersten bis vierzehnten Ausführungsform, wobei die Carbonylierung in einem Lösungsmittels, bevorzugt einem aprotischen Lösungsmittel durchgeführt.

In einer sechzehnten Ausführungsform betrifft die Erfindung ein Verfahren gemäß einer der ersten bis fünfzehnten Ausführungsform, wobei das Lösungsmittel eine oder mehrere Verbindung(en) ist und ausgewählt wird aus der Gruppe bestehend aus 4-Methyl-2-oxo-1,3-dioxolan, 1,3-Dioxolan-2-on, 2-Methyltetrahydrofuran, Tetrahydrofuran, 1,4-Dioxan, Dimethoxyethan (Glyme), Bis(2-methoxyethyl)ether (Diglyme), Tris(2-methoxyethyl)ether (Triglyme), Diethylether, Ethylacetat, Methylethylketon, *N*-Methylpyrrolidon, Acetonitril, Sulfolan, Dimethylsulfoxid, Dimethylformamid, Hexan, Toluol, Xylol, Ethylbenzol, Dichlorbenzol, und Chlorbenzol, bevorzugt 4-Methyl-2-oxo-1,3-dioxolan, 1,3-Dioxolan-2-on, 2-Methyltetrahydrofuran, Tetrahydrofuran, 1,4-Dioxan, Dimethoxyethan (Glyme), Bis(2-methoxyethyl)ether (Diglyme), Diethylether, Ethylacetat, Acetonitril, Toluol, Dichlorbenzol und Chlorbenzol, besonders bevorzugt Tetrahydrofuran.

In einer sechzehnten Ausführungsform betrifft die Erfindung ein Verfahren gemäß einer der ersten bis fünfzehnten Ausführungsform, wobei die Konzentration des Epoxids im Lösungsmittel von 0,5 mol/L bis 10,0 mol/L, bevorzugt von 1,0 mol/L bis 9,0 mol/L und besonders bevorzugt von 2,0 mol/L bis 8,0 mol/L beträgt.

In siebzehnten Ausführungsform betrifft die Erfindung ein Carbonylierungsproduktgemisch erhältlich nach dem erfindungsgemäßen Verfahren gemäß einer der ersten bis sechzehnten Ausführungsform, wobei der Anteil an cyclischem Anhydrid weniger als 99,0 mol-% bevorzugt von 80,0 bis 98,8 mol-% sowie der Anteil an polymeren Carbonylierungsprodukten weniger als 7,0 mol-% bevorzugt weniger als 2,0 mol-% beträgt, wobei die Stoffmengenanteile des cyclischen Anhydrids sowie der polymeren Carbonylierungsprodukte mittels der im Experimentalteil offenbarten Methode ¹H NMR Spektroskopie bestimmt wurde.

In achtzehnten Ausführungsform betrifft die Erfindung ein Verfahren zur Herstellung von Carbonylierungsfolgeprodukten, durch Umsetzung des Carbonylierungsproduktgemisches gemäß der siebzehnten Ausführungsform mit Glycolen und/oder Epoxiden gemäß der zweiten oder dritten Ausführungsform.

In neunzehnten Ausführungsform betrifft die Erfindung die Verwendung des erfindungsgemäßen Katalysatorsystems gemäß einer der ersten bis achtzehnten Ausführungsform für die Carbonylierung von Epoxiden.

### Beispiele

### Verwendete Ausgangsstoffe:

Argon, 4.8 Spectra, kurz Ar, 99.998%, Westfalen AG
Deuteriertes Tetrahydrofuran, THF-d₈, 99,5 %, Sigma Aldrich
1,2-Butylenoxid (>99%, Merck KGaA)
Dichlormethan, 99,8%, Sigma Aldrich
Diethylaluminiumchlorid, 0.9 M in Toluol, fisher scientific
Dicobaltoctacarbonyl, ≥90% (1-10% Hexan), Sigma Aldrich
1,2-Hexylenoxid (>96%, TCI Europe N.V.)
NaOH, 98-100%, Sigma Aldrich
THF, 99,9%, Sigma Aldrich
o-Phenylendiamin, 99%, Merck KGaA
Nickelacetat Tetrahydrat, 98%, Sigma Aldrich Chemie GmbH
Acetylaceton, 99,5%, Sigma Aldrich Chemie GmbH
HCl [g], 99,8%, Linde Gas
Triethylamin, 99%, Merck KGaA
Toluol, 99,8% Acros Organics
Butanol, 99,4% Sigma Aldrich Chemie GmbH
Methanol, 99,9%, Sigma Aldrich Chemie GmbH

Die Chemikalien wurden ohne weitere Aufreinigung für die Synthese eingesetzt.

### Carbonylierungsreaktion:

THF, 99,9%, Sigma Aldrich
EO, 99,9%, GHC
CO, 99.997%, Linde
Naphthalin, 99%, Sigma Aldrich

Alle Chemikalien wurden so eingesetzt wie sie erhalten wurden.

### IR-Analytik:

Alle IR-Messungen wurden auf einem Bruker Alpha-PFT-IR Spektrometer durchgeführt. Falls nötig wurden die Messungen unter Ar-Schutzgas durchgeführt. Für alle gemessenen Spektren wurde eine automatische Basislinienkorrektur vorgenommen.

### NMR-Analytik:

NMR-Spektren wurden auf einem Bruker AV400 oder AV300 oder Bruker AV600 Spektrometer bei Raumtemperatur aufgenommen. ¹H NMR Spektren wurden auf das Restprotonensignal des Lösungsmittels referenziert. Naphthalin diente als interner Standard.

### Katalysatorsynthese

### Katalysatorsystem 1: [(tmtaa)Al(THF)₂][Co(CO)₄] (erfindungsgemäß)

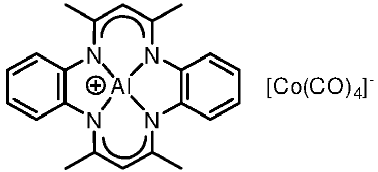

### Stufe 1: Ni-(tmtaa): (MW: 401,13 g/mol)

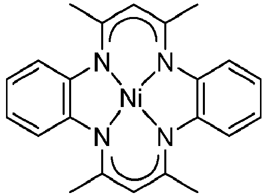

In einem 12,5 L Planschliffreaktor werden o-Phenylenediamin (478 g, 4,42 mol), Ni(OAc)₂ · 4 H₂O (549 g, 2,21 mol), Acetylaceton (442 g, 4,42 mol) vorgelegt und mit 5,4 L 1-Butanol versetzt. Die Suspension wird bei 130 °C für 3 h refluxiert. Anschließend wird die Reaktionsmischung auf Raumtemperatur abgekühlt und 1,1 L MeOH werden zugefügt. Anschließend wird die Mischung für 1 h auf -5 °C gekühlt. Der ausfallende violette Feststoff wird über ein Filtertuch abfiltriert und mit 3 x 450 mL kaltem MeOH (0-5 °C) gewaschen. Der Feststoff wird bei Umgebungsdruck/-temperatur getrocknet.

### Stufe 2 (tmtaa-H₂) · 2 HCl: (MW: 417,37 g/mol)

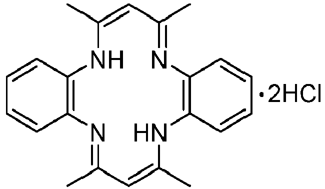

In einem 12,5 L Planschliffreaktor werden Ni(tmtaa) (753 g, 1,88 mol) und MeOH (7,6 L) gegeben. Durch die Suspension wird unter Rühren bei Raumtemperatur für 4 h Stunden HCl-Gas eingeleitet (300 L/h). Anschließend wird die Reaktionsmischung auf 0 °C abgekühlt und der Feststoff abfiltriert. Der weiße Feststoff wird mit kaltem MeOH (0-5°C, 3 x 500 mL) gewaschen, bis das Produkt farblos ist. Der Feststoff wird bei Umgebungsdruck/-temperatur getrocknet.

### Stufe 3 (tmtaa-H₂): (MW: 344,45 g/mol)

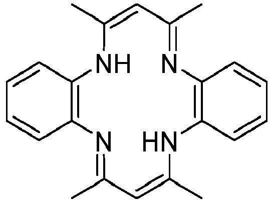

In einem 12,5 L Planschliffreaktor wird (tmtaa-H₂) · 2 HCl (713 g, 1,71 mol) vorgelegt und mit MeOH (8,20 L) versetzt. Zu der Suspension wird Triethylamin zugetropft bis die Reaktionsmischung einen pH-Wert von 9 erreicht. Der ausgefallene Feststoff wird abfiltriert und mit kaltem MeOH (0-5 °C) gewaschen (3 x 450 mL). Der Feststoff wird über Nacht unter vermindertem Druck und Umgebungstemperatur getrocknet.

### Stufe 4: (tmtaa)AlCl: (MW: 343,75 g/mol)

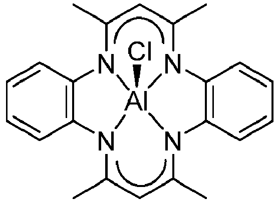

In einem 12,5 L Planschliffreaktor wird der freie Ligand tmtaa-H₂ (150 g, 436 mmol) unter Schutzgasatmosphäre in Dichlormethan (3,1 L) gelöst. Bei Raumtemperatur wird zur Lösung eine Et₂AlCl-Lösung in Toluol (0,9 M, 484 mL, 436 mmol) tropfenweise zugegeben (8 mL/min). Nach Zugabe der Lösung wird der Tropftrichter mit DCM (2 x 50 mL) gespült. Die Suspension wird für 3-4 h gerührt. Anschließend wird die Reaktionsmischung auf ¼ des Volumens eingeengt.

### Stufe 5: NaCo(CO)4: 193,96 g/mol

Unter Schutzgas werden 82,8 g Co₂(CO)8 (0,242 mol) in einem 2 L-Planschliffgefäß vorgelegt, mit 1,65 L THF versetzt und unter Rühren auf 0 °C gebracht. Anschließend erfolgt die Zugabe von NaOH-Pulver (69,3 g, 2,5 mol) unter Schutzgasgegenstrom und Rühren. Das erhaltene Reaktionsgemisch wird nun unter Lichtausschluss für 4 h bei 0 °C intensiv gerührt. Anschließend wird mit ca. 10 °C pro Stunde auf Raumtemperatur erwärmt. Der Feststoffanteil sedimentiert. Die überstehende Lösung wird zu der (tmtaa)AlCl-Lösung zugegeben (Nachspülen mit THF 2 x 1,1 L).

### Stufe 6: [(tmtaa)Al(THF)2]Co(CO)4: 688,65 g/mol

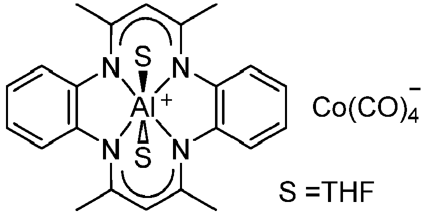

Das Reaktionsgemisch aus (tmtaa)AlCl-Lösung (Stufe 4) und NaCo(CO)₄-Lösung (Stufe 5) wird für 16 h gerührt. Anschließend wird das Reaktionsgemisch über eine Fritte filtriert. Unter vermindertem Druck wird das Lösungsmittel entfernt und der [(tmtaa)Al(THF)₂][Co(CO)₄] Komplex isoliert.

### Katalysatorsystem 2: [Cl(TPP)Al(THF)₂]⁺[Co(CO)₄]⁻ (Bis(tetra-hydrofuran)-meso-tetra(4-chlorophenyl)-porphyrinato-aluminium-cobalttetracarbonyl) (Vergleich)

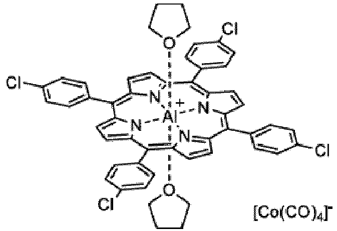

Die Synthese des literaturbekannten Katalysatorsystems 2 erfolgte gemäß der Versuchsbeschreibung auf S. 4958 in J. M. Rowley, E. B. Lobkovsky, G. W. Coates, J. Am. Chem. Soc. 2007, 129, 4948-4960.

### Katalysatorsystem 3: [(salph)Al(thf)₂]⁺[Co(CO)₄]⁻ N,N'-Bis(3,5-di-tert-butylsalicyliden)-1,2-phenylendiamino-aluminiumcobalt-tetracarbonyl (Vergleich)

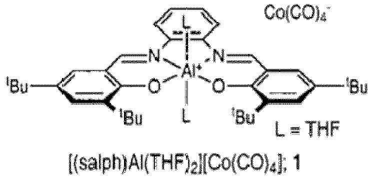

Die Synthese des literaturbekannten Katalysatorsystems 3 erfolgte gemäß der Versuchsbeschreibung in Getzler, Y. D. Y. L.; Mahadevan, V.; Lobkovsky, E. B.; Coates, G. W. J. Am. Chem. Soc. 2002, 124, 1174-1175.

### Carbonylierungsreaktion

### Beispiel 1

Unter inerten Bedingungen wird THF in einen 300 mL Stahlautoklav vorgelegt (40 mL) und eine Lösung von Katalysator 1 in THF (10 mL; 0,1 M) zugegeben. Der Reaktor wird auf 60 °C erwärmt und bei 3 barü N₂ auf 60 bar mit CO beaufschlagt. Es wird EO zudosiert (4,4 g, 0,10 mol). Die Reaktion wird bei konstantem CO-Druck (nachregulieren mit CO) für 3 h gerührt. Anschließend wird der Reaktor auf Raumtemperatur abgekühlt und der Druck abgelassen. Die Reaktionsmischung wird mittels ¹H NMR Spektroskopie in Gegenwart von Naphthalin als internem Standard analysiert (Tabelle 2, Eintrag 1).

### Beispiel 2

Unter inerten Bedingungen wird THF in einen 300 mL Stahlautoklav vorgelegt (40 mL) und eine Lösung von Katalysator 2 in THF (10 mL; 0,1 M) zugegeben. Der Reaktor wird auf 60 °C erwärmt und bei 3 barü N₂ auf 60 bar mit CO beaufschlagt. Es wird EO zudosiert (4,4 g, 0,10 mol). Die Reaktion wird bei konstantem CO-Druck (nachregulieren mit CO) für 3 h gerührt. Anschließend wird der Reaktor auf Raumtemperatur abgekühlt und der Druck abgelassen. Die Reaktionsmischung wird mittels ¹H NMR Spektroskopie in Gegenwart von Naphthalin als internem Standard analysiert (Tabelle 2, Eintrag 2).

### Beispiel 3

Unter inerten Bedingungen wird THF in einen 300 mL Stahlautoklav vorgelegt (40 mL) und eine Lösung von Katalysator 1 in THF (10 mL; 0,1 M) zugegeben. Der Reaktor wird auf 60 °C erwärmt und bei 3 barü N₂ auf 60 bar mit CO beaufschlagt. Es wird EO zudosiert (4,4 g, 0,10 mol). Die Reaktion wird bei konstantem CO-Druck (nachregulieren mit CO) für 3 h gerührt. Anschließend wird die Temperatur auf 100 °C erhöht und bei einem konstanten Druck von 60 bar für weitere 1,5 h gerührt. Der Reaktor wird auf Raumtemperatur abgekühlt und der Druck abgelassen. Die Reaktionsmischung wird mittels ¹H NMR Spektroskopie in Gegenwart von Naphthalin als internem Standard analysiert (Tabelle, Eintrag 3).

### Beispiel 4

Unter inerten Bedingungen wird THF in einen 300 mL Stahlautoklav vorgelegt (40 mL) und eine Lösung von Katalysator 2 in THF (10 mL; 0,1 M) zugegeben. Der Reaktor wird auf 60 °C erwärmt und bei 3 barü N₂ auf 60 bar mit CO beaufschlagt. Es wird EO zudosiert (4,4 g, 0,10 mol). Die Reaktion wird bei konstantem CO-Druck (nachregulieren mit CO) für 3 h gerührt. Anschließend wird die Temperatur auf 100 °C erhöht und bei einem konstanten Druck von 60 bar für weitere 1,5 h gerührt. Der Reaktor wird auf Raumtemperatur abgekühlt und der Druck abgelassen. Die Reaktionsmischung wird mittels ¹H NMR Spektroskopie in Gegenwart von Naphthalin als internem Standard analysiert (Tabelle, Eintrag 4).

### Beispiel 5

Unter inerten Bedingungen wird THF in einen 300 mL Stahlautoklav vorgelegt (20 mL) und eine Lösung von Katalysator 1 in THF (30 mL; 0,1 M) zugegeben. Der Reaktor wird auf 60 °C erwärmt und bei 3 barü N₂ auf 60 bar mit CO beaufschlagt. Es wird EO zudosiert (13,2 g, 0,30 mol). Die Reaktion wird bei konstantem CO-Druck (nachregulieren mit CO) für weitere 3 h gerührt. Anschließend wird die Temperatur auf 110 °C erhöht und bei einem konstanten Druck von 60 bar für weitere 2 h gerührt. Der Reaktor wird auf Raumtemperatur abgekühlt und der Druck abgelassen. Die Reaktionsmischung wird mittels ¹H NMR Spektroskopie in Gegenwart von Naphthalin als internem Standard analysiert (Tabelle, Eintrag 5).

### Beispiel 6

Unter inerten Bedingungen wird THF in einen 300 mL Stahlautoklav vorgelegt (20 mL) und eine Lösung von Katalysator 2 in THF (30 mL; 0,1 M) zugegeben. Der Reaktor wird auf 60 °C erwärmt und bei 3 barü N₂ auf 60 bar mit CO beaufschlagt. Es wird EO zudosiert (13,2 g, 0,30 mol). Die Reaktion wird bei konstantem CO-Druck (nachregulieren mit CO) für weitere 3 h gerührt. Anschließend wird die Temperatur auf 110 °C erhöht und bei einem konstanten Druck von 60 bar für weitere 2 h gerührt. Der Reaktor wird auf Raumtemperatur abgekühlt und der Druck abgelassen. Die Reaktionsmischung wird mittels ¹H NMR Spektroskopie in Gegenwart von Naphthalin als internem Standard analysiert (Tabelle, Eintrag 6).

### Beispiel 7-8

In einem Schlenkkolben mit Katalysator (0,12 mmol, Bsp 7: Kat 1; Bsp. 8: Kat 2) werden unter Ar-Atmosphäre wasserfreies THF (1,16 mL) gefolgt von PO (0,84 mL, 12 mmol) zugegeben. Anschließend wird die Mischung in einen 10 mL-Stahlautoklaven überführt. Der Reaktor wird mit 60 bar bedruckt und das Reaktionsgemisch für 4 h bei 60 °C gerührt. Die Reaktion wird weitere 6 h bei 110 °C und 60 bar fortgeführt. Am Ende wird der Reaktor abgekühlt und der Druck abgelassen. Die Reaktionsmischung wird mittels ¹H NMR Spektroskopie in Gegenwart von Naphthalin als internem Standard analysiert.

### Beispiel 9-10

In einem Schlenkkolben mit Katalysator (0,12 mmol, Bsp 9: Kat 1; Bsp. 10: Kat 2) werden unter Ar-Atmosphäre wasserfreies THF (1,16 mL) gefolgt von PO (0,84 mL, 12 mmol) zugegeben. Anschließend wird die Mischung in einen 10 mL-Stahlautoklaven überführt. Der Reaktor wird mit 60 bar bedruckt und das Reaktionsgemisch für 4 h bei 60 °C gerührt. Die Reaktion wird weitere 6 h bei 110 °C und 20 bar fortgeführt. Am Ende wird der Reaktor abgekühlt und der Druck abgelassen. Die Reaktionsmischung wird mittels ¹H NMR Spektroskopie in Gegenwart von Naphthalin als internem Standard analysiert.

### Beispiel 11-12

In einem 5 mL Glasgefäß mit Septum wird der Katalysator (0,03 mmol; Bsp 11: Kat 1; Bsp. 12: Kat 2) in trockenem THF (3 mL) unter inerten Bedingungen gelöst. Anschließend wird die Katalysatorlösung bei kontrollierten Bedingungen unter Rühren der Luftatmosphäre ausgesetzt. Hierfür wird das Septum mit zwei Kanülen mit je 0,4 mm Durchmesser angestochen. Die Katalysatorlösung wird für 28 h unter den genannten Bedingungen intensiv mittels Magentrührer gerührt. Anschließend wird die Katalysatorlösung für eine Carbonylierungsreaktion von Epoxiden verwendet. Hierfür werden 0,65 mL der Katalysatorlösung (0,0065 mmol) und 45 µL Propylenoxid (0,93 mmol) in einen 10 mL- Stahlautoklaven transferiert und mit Ar (1 bar) gespült. Anschließend wird auf 60 bar CO beaufschlagt und der Reaktor auf 60 °C für 4 h erhitzt. Es wird für weitere 2 h bei 110 °C und 60 bar gerührt. Am Ende wird der Reaktor abgekühlt und der Druck abgelassen. Die Reaktionsmischung wird mittels ¹H NMR Spektroskopie in Gegenwart von Naphthalin als internem Standard analysiert.

### Beispiel 13-14

Der Katalysator wird als Feststoff 28 h einer Luftatmosphäre ausgesetzt. Anschließend wird dieser in einem Schlenkkolben eingewogen (0,04 mmol, Bsp 13: Kat 1; Bsp. 14: Kat 2) und es wird unter Ar-Atmosphäre wasserfreies THF (1,72 mL) gefolgt von PO (0,28 mL, 4 mmol) zugegeben. Anschließend wird die Mischung in einen 10 mL-Stahlautoklaven überführt. Der Reaktor wird mit 60 bar bedruckt und das Reaktionsgemisch für 4 h bei 60 °C gerührt. Die Reaktion wird weitere 2 h bei 110 °C und 60 bar weitergeführt. Am Ende wird der Reaktor abgekühlt und der Druck abgelassen. Die Reaktionsmischung wird mittels ¹H NMR Spektroskopie in Gegenwart von Naphthalin als internem Standard analysiert.

### Beispiel 15

In einem Schlenkkolben mit Katalysator (0,02 mmol, Kat 1) werden unter Ar-Atmosphäre wasserfreies THF (2,0 mL) gefolgt von 1,2-Epoxybutan (0,17 mL, 2 mmol) zugegeben. Anschließend wird die Mischung in einen 10 mL-Stahlautoklaven überführt. Der Reaktor wird mit 60 bar bedruckt und das Reaktionsgemisch für 4 h bei 60 °C gerührt. Die Reaktion wird weitere 4 h bei 110 °C und 60 bar fortgeführt. Am Ende wird der Reaktor abgekühlt und der Druck abgelassen. Die Reaktionsmischung wird mittels ¹H NMR Spektroskopie in Gegenwart von Naphthalin als internem Standard analysiert.

### Beispiel 16

In einem Schlenkkolben mit Katalysator (0,02 mmol, Kat 1) werden unter Ar-Atmosphäre wasserfreies THF (2,0 mL) gefolgt von 1,2-Epoxyhexan (0,24 mL, 2 mmol) zugegeben. Anschließend wird die Mischung in einen 10 mL-Stahlautoklaven überführt. Der Reaktor wird mit 60 bar bedruckt und das Reaktionsgemisch für 4 h bei 60 °C gerührt. Die Reaktion wird weitere 5 h bei 110 °C und 55 bar weitergeführt. Am Ende wird der Reaktor abgekühlt und der Druck abgelassen. Die Reaktionsmischung wird mittels ¹H NMR Spektroskopie in Gegenwart von Naphthalin als internem Standard analysiert.

**Tabelle 1: Ausführungsbeispiele zur Carbonylierung von Epoxiden**

| **Bsp.** | **Kat.** | **Kat. [mol-%]** | **Epoxid^{[a]}** | **Epoxid [mol/L]** | **p_{CO} [bar]** | **t [h]** | **T [°C]** | **β-Lacton [mol-%]** | **Cycl. Anh. [mol-%]** | **Uml.p.^{[b]} [mol-%]** | **pol. NP^{[c]} [mol-%]** |
|---|---|---|---|---|---|---|---|---|---|---|---|
| 1 (Vgl.) | 1 | 1 | EO | 2,0 | 57 | 3 | 60 | 88,0 | 12,0 | - | - |
| 2 (Vgl.) | 2 | 1 | EO | 2,0 | 57 | 3 | 60 | 30,0 | 66,6 | 1,2 | 1,8 |
| 3 | 1 | 1 | EO | 2,0 | 57/57 | 3/1.5 | 60/100 | - | 98,7 | 0,3 | 0,2 |
| 4 (Vgl.) | 2 | 1 | EO | 2,0 | 57/57 | 3/1.5 | 60/100 | 1,0 | 93,4 | 2,8 | 2,8 |
| 5 | 1 | 1 | EO | 6,0 | 57/57 | 4/2 | 60/110 | 0,3 | 97,0 | 1,2 | 1,5 |
| 6 (Vgl.) | 2 | 1 | EO | 6,0 | 57/57 | 4/2 | 60/110 | 0 | 86,2 | 2,8 | 11 |
| 7 | 1 | 1 | PO | 6,0 | 60/60 | 4/6 | 60/100 | 53 | 41 | 1 | 5 |
| 8 (Vgl.) | 2 | 1 | PO | 6,0 | 60/60 | 4/6 | 60/100 | 0 | 48 | 2 | 44 |
| 9 | 1 | 1 | PO | 6,0 | 60/20 | 4/6 | 60/110 | 35 | 58 | 1 | 6 |
| 10 (Vgl.) | 2 | 1 | PO | 6,0 | 60/20 | 4/6 | 60/110 | - | 70 | 2 | 28 |
| 11 | 1^{[d]} | 0,7 | PO | 1,4 | 60/60 | 4/2 | 60/110 | 58 | 0 | 15 | 9 |
| 12 (Vgl.) | 2^{[d]} | 0,7 | PO | 1,4 | 60/60 | 4/2 | 60/110 | 0 | 0 | 0 | 44^{[e]} |
| 13 | 1^{[f]} | 1 | PO | 2,0 | 60/60 | 4/2 | 60/110 | 90 | 0 | 4 | 6 |
| 14 (Vgl.) | 2^{[f]} | 1 | PO | 2,0 | 60/60 | 4/2 | 60/110 | 0 | 0 | 16 | 16^{[e]} |
| 15 | 1 | 1 | BO | 0,9 | 60/55 | 4/4 | 60/110 | 9 | 91 | - | - |
| 16 | 1 | 1 | HO | 0,9 | 60/55 | 4/5 | 60/110 | - | 98 | - | - |

| | | | | | | | | | | | |
|---|---|---|---|---|---|---|---|---|---|---|---|
| ^{[a]} EO: Ethylenoxid; PO: Propylenoxid; BO: 1,2-Butylenoxid; HO: 1,2-Hexylenoxid. ^{[b]} Umlagerungsprodukte (Acetaldehyd, Propanal, Aceton, Acrylsäure, Crotonsäure); ^{[c]} polymere Nebenprodukte (Polyether/-ester); ^{[d]}Katalysatorlösung wurde vor Verwendung 28 h einer Luftatmosphäre ausgesetzt; ^{[e]}Polymere Nebenproduktfraktion ist ein reiner Polyether ohne Hinweis auf Esterstrukturen als Folge von Carbonylierung; ^{[f]}Katalysator als Feststoff wurde vor Verwendung 28 h einer Luftatmosphäre ausgesetzt. | | | | | | | | | | | |

**Tabelle 2: Katalysator-Stabilitätsmessung mittels IR-Analytik der jeweiligen "Co(CO)"-Bande.^{a}**

| Zeit [h] | Katalysatorsystem 1: FT-IR "Co(CO)" (bei 1882 cm⁻¹) | Katalysatorsystem 2 (Vergleich): FT-IR "Co(CO)" (bei 1874 cm⁻¹) | Katalysatorsystem 3 (Vergleich): FT-IR "Co(CO)" (bei 1866 cm⁻¹) |
|---|---|---|---|
| 0 | 100% | 100% | 100 % |
| 0,10 | 95,9 % | 68,2 % | 36,1 % |
| 0,98 | 67,6 % | 62,5 % | 6,7 % |
| 2,00 | 64,7 % | 50,2 % | 5,0 % |
| 4,13 | 56,9 % | 26,5 % | 0 % |
| 5,03 | 56,8 % | 10,0 % | - |
| 24 | 23,8 % | 0 % | - |

| | | | |
|---|---|---|---|
| *^{a}* Analyse der Katalysatoren als Feststoffe mittels IR-Analytik zu unterschiedlichen Zeitpunkten der Exposition an Luft. Die prozentuale Abnahme ergibt sich aus dem Intensitätsverhältnis der "Co(CO)"-Bande bei FT IR-Messung zwischen t und t₀. | | | |

## Patentansprüche

1. Verfahren zur Carbonylierung von Epoxiden in Gegenwart von Kohlenmonoxid und eines Katalysatorsystems,
wobei das Katalysatorsystem eine Struktur der Formel (I) aufweist: 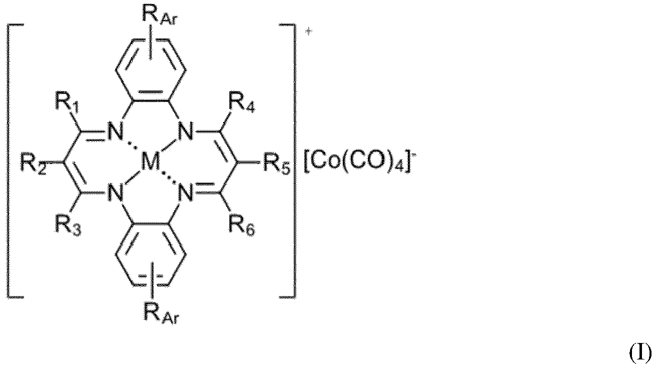 wobei M Al, Cr, Fe, Mo, W, Sc, Y, Bi, Ru, Rh, La, Ce, Nd, Sm, In oder Ga ist, wobei M bevorzugt Al oder Cr ist,
wobei R_{Ar}: an jeder Position unabhängig voneinander ausgewählt aus der Gruppe von: H, C1-C12-Alkyl, C2-C12-Alkenyl, C2-C12-Alkinyl, O-(C1-C12-Alkyl), O-(C2-C12-Alkenyl), O-(C2-C12-Alkinyl), O-CO-(C1-C12-Alkyl), O-CO-(C2-C12-Alkenyl), O-CO-(C2-C12-Alkinyl), -CH2-O-(C1-C12-Alkyl), -CH2-O-(C2-C12-Alkenyl), -CH2-O-(C2-C12-Alkinyl), -CO-O-(C1-C12-Alkyl), -CO-O-(C2-C12-Alkenyl), -CO-O-(C2-C12-Alkinyl), -CF₃, -OCF₃, bevorzugt H, Methyl, Ethyl, n-Propyl, iso-Propyl, n-Butyl-, iso-Butyl, tert-Butyl, O-Methyl, O-Ethyl, O-n-Propyl, O-iso-Propyl, O-n-Butyl-, O-iso-Butyl, O-tert-Butyl, besonders bevorzugt H oder Methyl,
wobei R₁, R₃, R₄, R₆ an jeder Position unabhängig voneinander ausgewählt aus der Gruppe von H, C1-C12-Alkyl, C2-C12-Alkenyl, C2-C12-Alkinyl, Aryl (C6R'5; R' an jeder Position unabhängig voneinander gewählt aus der Gruppe von H, C1-C12-Alkyl), Phenyl, Benzyl, Cyclohexyl (C6R'11; R' an jeder Position unabhängig voneinander gewählt aus der Gruppe von H, C1-C12-Alkyl), -CF₃, bevorzugt Methyl, Ethyl, , n-Propyl, iso-Propyl, n-Butyl-, iso-Butyl, tert-Butyl, -CF₃, Phenyl, besonders bevorzugt Methyl und tert-Butyl,
wobei R₂, R₅ an jeder Position unabhängig voneinander ausgewählt aus der Gruppe von H, C1-C12-Alkyl, C2-C12-Alkenyl, C2-C12-Alkinyl, Aryl (C6R'5; R' an jeder Position unabhängig voneinander gewählt aus der Gruppe von H, C1-C12-Alkyl), Phenyl, Benzyl, Cyclohexyl (C6R'11; R' an jeder Position unabhängig voneinander gewählt aus der Gruppe von H, C1-C12-Alkyl), -CF₃, bevorzugt H, Methyl, Ethyl, n-Propyl, iso-Propyl, n-Butyl-, iso-Butyl, tert-Butyl, -CF₃, Phenyl, besonders bevorzugt H,
wobei die Carbonylierung bei Temperaturen von 40 °C bis 130 °C, bevorzugt von 45 °C bis 125 °C und besonders bevorzugt von 50 °C bis 120 °C durchgeführt wird,
wobei die Carbonylierung folgende Schritte umfasst
i) Umsetzung des Epoxids mit Kohlenmonoxid bei einer ersten Temperatur T(i) zu einem Lacton-haltigen Intermediat-Gemisch (i)
ii) Umsetzung des Lacton-haltigen Intermediat-Gemischs (i) mit Kohlenmonoxid bei einer zweiten Temperatur T(ii) zum Produktgemisch,
wobei T(ii) > T(i) ist.

2. Verfahren gemäß Anspruch 1, wobei M in Formel (I) Aluminium (Al) ist.

3. Verfahren gemäß Anspruch 1 oder 2, wobei das Katalysatorsystem Strukturen der Formel (II), (III), (IV), (V), (VI) und/oder (VII) aufweist: 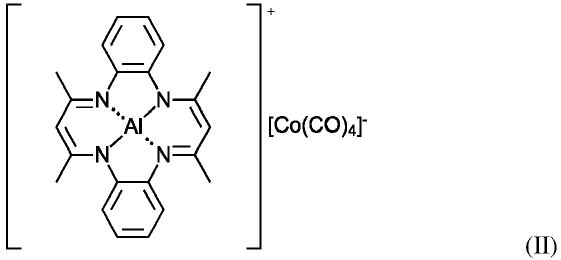 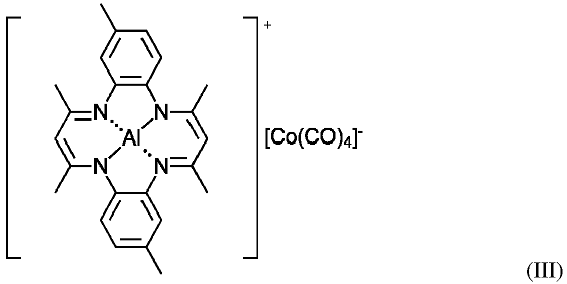 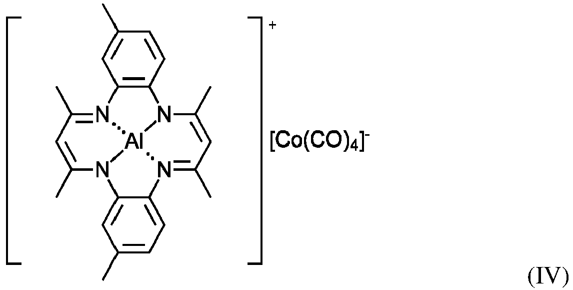 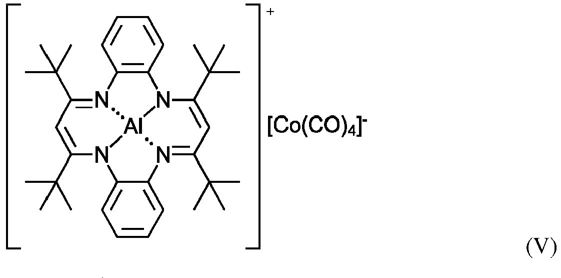 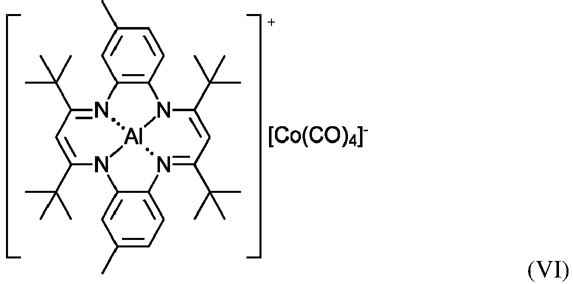 und/oder

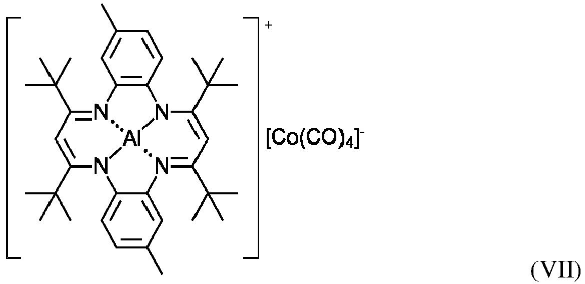

4. Verfahren gemäß einem der Ansprüche 1 bis 3, wobei T(i) von 40 °C bis 80 °C und T(ii) größer 80°C bis 130 °C, bevorzugt T(i) von 45 °C bis 80 °C und T(ii) größer 80°C bis 125 °C und besonders bevorzugt T(i) von 50 °C bis 80 °C und T(ii) größer 80°C bis 120 °C ist.

5. Verfahren gemäß einem der Ansprüche 1 bis 4, wobei die Carbonylierung bei Drücken von 1 bara bis 120 bara, bevorzugt von 10 bara bis 100 bara und besonders bevorzugt von 20 bara bis 80 bara durchgeführt wird, wobei Schritt i) bei einem Druck p(i) und Schritt ii) bei einem Druck p(ii) durchgeführt wird, wobei p(i) bevorzugt größer als p(ii) ist.

6. Verfahren gemäß einem der Ansprüche 1 bis 5, wobei die Carbonylierung in einem Lösungsmittel durchgeführt wird.

7. Verfahren gemäß Anspruch 6, wobei das Lösungsmittel eine oder mehrere Verbindung(en) ist und ausgewählt wird aus der Gruppe bestehend aus 4-Methyl-2-oxo-1,3-dioxolan, 1,3-Dioxolan-2-on, 2-Methyltetrahydrofuran, Tetrahydrofuran, 1,4-Dioxan, Dimethoxyethan (Glyme), Bis(2-methoxyethyl)ether (Diglyme), Tris(2-methoxyethyl)ether (Triglyme), Diethylether, Ethylacetat, Methylethylketon, *N*-Methylpyrrolidon, Acetonitril, Sulfolan, Dimethylsulfoxid, Dimethylformamid, Hexan, Toluol, Xylol, Ethylbenzol, Dichlorbenzol, und Chlorbenzol, bevorzugt 4-Methyl-2-oxo-1,3-dioxolan, 1,3-Dioxolan-2-on, 2-Methyltetrahydrofuran, Tetrahydrofuran, 1,4-Dioxan, Dimethoxyethan (Glyme), Bis(2-methoxyethyl)ether (Diglyme), Diethylether, Ethylacetat, Acetonitril, Toluol, Dichlorbenzol und Chlorbenzol, besonders bevorzugt Tetrahydrofuran.

8. Verfahren gemäß einem der Ansprüche 1 bis 7, wobei das Epoxid Ethylenoxid, Propylenoxid, 1,2-Butylenoxid, 1,2-Pentylenoxid, 1,2-Hexylenoxid, 1,2-Heptylenoxid und/oder 1,2-Octylenoxid, bevorzugt Ethylenoxid und/oder Propylenoxid ist.

9. Verfahren gemäß einem der Ansprüche 6 bis 8, wobei die Konzentration des Epoxids im Lösungsmittel von 0,5 mol/L bis 10,0 mol/L bevorzugt von 1,0 mol/L bis 9,0 mol/L und besonders bevorzugt von 2,0 mol/L bis 8,0 mol/L beträgt.

10. Verfahren gemäß einem der Ansprüche 1 bis 9, wobei das Katalysatorsystem in Mengen von 0,10 mol-% bis 10,00 mol-% bevorzugt von 0,25 mol-% bis 5,00 mol-% und besonders bevorzugt von 0,50 mol-% bis 2,00 mol-% bezogen auf das Epoxid eingesetzt wird.

11. Verfahren gemäß einem der Ansprüche 1 bis 10, wobei das Lacton-haltige Intermediat-Gemisch (i) eine Selektivität zur Bildung des Lactons von 50 % bis 100 % bevorzugt von 50 % bis 95 % und besonders bevorzugt von 60 % bis 90 % aufweist.

12. Carbonylierungsproduktgemisch erhältlich nach mindestens einem der Ansprüche 1 bis 11, wobei der Anteil an cyclischen Anhydrids weniger als 99,0 mol-% bevorzugt von 80,0 bis 98,8 mol-% sowie der Anteil an polymeren Carbonylierungsprodukten weniger als 7,0 mol-% bevorzugt weniger als 2,0 mol-% beträgt, wobei die Stoffmengenanteile des cyclischen Anhydrids sowie der polymeren Carbonylierungsprodukte mittels der im Experimentalteil offenbarten Methode ¹H NMR Spektroskopie bestimmt wurde.

13. Verfahren zur Herstellung von Carbonylierungsfolgeprodukten durch Umsetzung des Carbonylierungsproduktgemisches gemäß Anspruch 12 mit Glycolen und/oder Epoxiden.

14. Verwendung des Katalysatorsystems gemäß einem der Ansprüche 1 bis 13 zur Carbonylierung von Epoxiden.
